# EUROPEAN PATENT APPLICATION

(11) **EP 1 634 530 A1**
(43) Date of publication of application: **15.03.2006**
(21) Application number: 05019638.5
(22) Date of filing: 09.09.2005
(51) Int. Cl.: A61B 5/00

(54) **System and method for displaying information related to a physical parameter of an individual**

(30) Priority: 13.09.2004 EP 04405571
(71) Applicant: International Business Machines Corporation, Armonk, N.Y. 10504 (US)
(72) Inventor: Nidd, Mike, 8802 Kilchberg (CH); Husemann, Dirk, 8134 Adliswil (CH)
(74) Representative: Meyer, Michael Josef

(57) **Abstract**

A system and methods are disclosed for displaying information related to a physical parameter of an individual. Such system comprises a sensor (11) for measuring a physical parameter of an individual, a database (21) remote from the sensor (11) for storing data assigned to an individual, and a display (12, 32) remote from the database (21) for displaying the information which information is based on a value measured and provided by the sensor (11) for this individual and based on the data assigned to this individual and provided by the database (21).

## Description

### FIELD OF THE INVENTION

The invention relates to a system and methods for displaying information related to a physical parameter of an individual, and to a related sensor unit and a mobile hub.

### BACKGROUND OF THE INVENTION

Many health-related measurements with value as stand-alone readings, such as blood pressure, ear temperature, or body mass, are more useful when placed in the context of previous readings from the same person.

This is already known but is, at present, used in only two ways: Patient data such as measurement values are collected; then the values are transferred to a remote server for later analysis. This reflects normal hospital operation. Or: The values measured are recorded in the sensor itself, which sensor is used only by one patient, as with an ear-temperature thermometer or blood pressure meter that can graph its last ten readings.

Prior art related to an ear thermometer as an example for such a sensor for measuring a physical parameter of an individual is disclosed in http://www.braun.com/global/products/healthwellness/earthermometers/thermoscan/faq/accur acy.html, in http://www.infrared-thermometer.co.uk, or in http://nmij.jp/ishii200309.pdf, all of them retrieved and accessed on the Internet on July 14, 2004. Another IR thermometer is disclosed in US patent No. 5,871,279. Another type of health-related sensor, a peak flow meter, is disclosed in http://www.lungusa.org/asthma/astpeakflow.html, retrieved and accessed on the Internet on July 14, 2004.

As for the integration of a database storing multiple records of patient data into the medical evaluation process, on the Applicant's web site http://www.zunch.ibm.com/news/03/mobilehealth.html, accessed on the Internet on November 11, 2003, a system is disclosed comprising a blood pressure monitor and a patient compliance device, both connected via Bluetooth to a cell phone serving as a mobile hub, and to a laptop computer. Patient values measured by the blood pressure monitor are transmitted.

According to http://www.haifa.ibm.com/projects/software/foak/mhealth.html, retrieved and accessed on the Internet on November 13, 2003, there is provided an application that offers physicians the ability to see updated records for their patients. All electronic patient records are stored on a server which can be accessed by the physician's mobile device.

Clearly, medical sensors have the need for individual calibration. It is useful for such devices when used by a single patient, to help establish this calibration by storing a history of measurements. On the other hand, for a medical professional, it is not usually practical to provide and carry a collection of different sensors for each patient. As for having only one sensor applied to many patients, storage on the sensor of reading histories would be useless. unless the sensor could know which patient was evaluated, and could sort these values internally. Furthermore, if an instrument were to be replaced, the calibration data for all possible patients would have to be uploaded to the new instrument.

However, today there is no chance for a medical personnel to use a medical sensor unit as generalized device applied to many different patients and at the same time being informed not only about the value currently measured but also on data specific to the patient, e.g. in order to better assess the measured value from a medical view.

### SUMMARY OF THE INVENTION

According to one aspect of the present invention, there is provided a system for displaying information related to a physical parameter of an individual, the system comprising a sensor for measuring a physical parameter of an individual, a database remote from the sensor for storing data assigned to an individual, and a display remote from the database for displaying the information which information is based on a value measured and provided by the sensor for this individual and based on the data assigned to this individual and provided by the database.

Although the system is presented in terms of human medicine, it can be equally useful for veterinary readings. Thus, the term individual includes both human and animal.

The value is particularly in combining data analysis techniques to give sensor units comprising medical sensors better performance than their local storage or computing power would allow. On the other hand, this allows a single sensor to be similarly useful to an unlimited number of patients.

It is preferred to provide wireless connectivity between the database and the sensor, or if a mobile hub is introduced as referred to in one of the following preferred embodiments, between the sensor and the mobile hub, and between the mobile hub and the database. Wireless connectivity allows the benefits to be enjoyed for shared sensors, such as those carried around by doctors on patient visits.

The data accessed from the database can embody different content as long as it is specific to an individual. Typically, such database provides data specific to many different patients, e.g. if the database is implemented on a hospital server, and a function is provided for querying the database for individuals, identified by an identity code for example. Such data can be values measured by the sensor in the past in one preferred embodiment. According to another preferred embodiment, such data includes a correction value specific to an individual for correcting a value measured by the sensor at this individual. Such correction value in particular can be a calibration value.

The information to be displayed is based on the value measured and the data provided by the database. Thus, in one embodiment, the information comprises the value measured and provided by the sensor and the data provided by the database, e.g. next to each other on the joint display. This is in particular helpful to medical staff operating the display, if e.g. the data provided by the database are historical values measured, such that the medical staff can understand at one glance on the display the history of measured values as well as the value currently measured, and if necessary put the value currently measured into context with the values measured in the past.

However, the information displayed can also be result of a computation with the measured value and the stored data as input. E.g., if the data stored includes or represents a correction value specific to the individual, a corrected value can determined by processing - e.g. multiplying - the value measured at this individual with the correction value. The corrected value then represents the information to be displayed.

Any such computation is preferably performed by a control entity. A database unit comprising the database from which patient data are retrieved from preferably also comprises the control entity. However, also a mobile hub introduced below can comprise the control entity. In connection with this embodiment, it is an additional advantage that the mobile hub and/or the database unit - also referred to as back-end server - can offer computing assistance as well is storage, in particular when the sensor unit is not designed for doing so. For example, when sending data in form of a patient history to the sensor unit, the database unit might also have calculated and transmitted the expected body temperature or oxygen peak flow to the sensor unit, so the sensor unit can set "alarm levels" uniquely for each patient. Further, because the back-end server does not have the power and cost limitations of end devices like sensors, it can offer computing assistance in recognising dangecous patterns that the sensor itself would not have been able to identify.

In practice, where an intermediate device such as the mobile hub is used, some computing assistance may also be added by that intermediate device. However, in some specific embodiments, also a sensor unit comprising the sensor might additionally comprise the control entity.

As already addressed, another preferred embodiment comprises a mobile hub as intermediary device between the database and the sensor. This means that the sensor communicates with the mobile hub, and the mobile hub communicates with the database. The mobile hub is preferably embodied as mobile phone or handheld comprising wireless communication means, in particular a first wireless interface for communicating to the sensor, such as a WLAN or a Bluetooth interface, and a second wireless interface for communicating to the database, such as a GSM interface.

The display for displaying the information is remote from the database unit. So is the sensor. Unit in this context is a constructional object. Remote from a unit thus means not integral part of the unit. The display thus can be part of the sensor unit, or - if provided - part of the mobile hub.

Hence, many different locations can be used for arranging the display and the control entity if any.

Preferably, the system comprises means for determining the identity of an individual. This is in particular useful for learning which data shall be retrieved from the database in order to co-display or correlate such data with the value measured for the individual with this identify. Such means can be sophisticated sensors performing e.g. biometric measurements in order to determine the identity of an individual. On the other hand, such means can be as simple as it keyboard for entering the identity of the individual. Preferably, such means are part of the mobile hub or connected to it. This is in particular helpful, as once the mobile hub is made aware of the identity of the current patient, then it can send calibration - or other patient specific - data to the individual instruments connected to the mobile hub as they require it. The individual may be identified directly - e.g. by using a keyboard, handwriting recognition, voice-to-text transformer, face or other biometric recognition sensors, etc. to input the identity - or implicitly - e.g. by using GPS identification of a patient's home, a beacon signal, etc.

Other aspects of the invention refer to methods for displaying information related to a physical parameter of an individual. These methods in particular reflect steps executed by a sensor unit or a mobile hub as indicated in dependent claims, depending on the capabilities provided in such devices. The order of steps in each method can be interchanged where reasonable.

A first method comprises receiving data assigned to the individual from a remote database, receiving a value measured by a sensor for measuring a physical parameter of the individual, and displaying the information remote from the database, which information is based on the value received from the sensor and on the data received from the database.

A second method comprises receiving a value measured by a sensor for measuring a physical parameter of the individual, transmitting the value to a remote control entity, in response to transmitting the value to the control entity: receiving information which information is based on the value measured by the sensor and on data assigned to the individual and stored in a remote database, and displaying the information remote from the database.

A third method comprises receiving a value measured by a sensor for measuring a physical parameter of the individual, transmitting the value to a remote control entity, in response to transmitting the value to the control entity: receiving information which information is based on the value measured by the sensor and on data assigned to the individual and stored in a remote database, and transmitting the information to a display remote from the database.

A fourth method comprises receiving a value measured by a sensor for measuring a physical parameter of the individual, receiving data assigned to the individual from a remote database, determining the information which information is based on the value measured by the sensor and on data assigned to the individual and stored in a remote database, and transmitting the information to a display remote from the database.

According to another aspect of the present invention, there is provided a computer program element comprising program code for performing the steps of the any method as claimed when said program code is run on a processing unit.

According to a further aspect of the present invention, there is provided a sensor unit comprising a control entity designed for performing steps according to any method as claimed.

According to a last aspect of the present invention, there is provided a mobile hub comprising a control entity designed for performing steps according to a method as claimed.
Advantages of the methods, apparatus, the computer program element, and the embodiments go along with the advantages of the system and its embodiments as described above.

### BRIEF DESCRIPTION OF THE DRAWINGS

The invention and its embodiments will be more fully appreciated by reference to the following detailed description of presently preferred but nonetheless illustrative embodiments in accordance with the present invention when taken in conjunction with the accompanying drawings.

### The figures are illustrating:

FIG. 1 a diagram of a first system according to the present invention,
FIG. 2 a diagram of a second system according to the present invention,
FIG. 3 a flow chart representing a first method according to the present invention,
FIG- 4 a flow chart representing a second method according to the present invention, and
FIG. 5 a flow chart representing a third method according to the present invention,
FIG. 6 diagrams representing embodiments of the present invention, which diagrams indicate the location of functions to be performed as well as the exchange of data,
FIG. 7 diagrams representing embodiments of the present invention, which diagrams indicate the location of functions to be performed as well as the exchange of data, and
FIG. 8 different diagrams illustrating displays for displaying information according to embodiments of the present invention.

Different figures may contain identical references, representing elements with similar or uniform content.

### DETAILED DESCRIPTION OF THE DRAWINGS

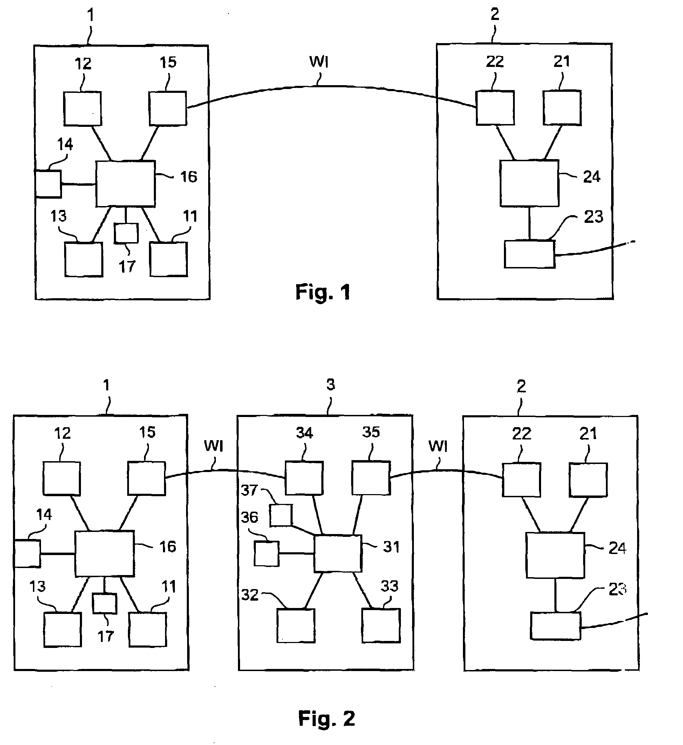

FIG. 1 illustrates a first system according to the present invention. The system comprises a sensor unit 1 and a database unit 2 remote from the sensor unit 1, i.e. the sensor unit 1 and the database unit 2 do not physically form a joint unit. The sensor unit 1 and the database unit 2 communicate via a wireless interface WI such as Bluetooth or wireless LAN. Accordingly, both of the units 1 and 2 comprise a wireless communications module 15 respectively 22 fosending and receiving wireless messages via the wireless interface WI.

The sensor unit 1 includes a sensor 11 for measuring a physical parameter of an individual. Such physical parameter can e.g. be the body temperature, blood pressure, pulse. Accordingly, the sensor 11 and also the sensor unit 1 can also be referred to as thermometer, blood pressure sensor, etc., whichever physical parameter the sensor is determined to measure. The sensor unit 1 is a mobile unit and typically can be reused on multiple individuals. As being embodied as a mobile unit, a battery 13 is provided for supplying the sensor unit 1 with electrical energy.

A measurement can be started by way of pushing a start button 14. Pushing the start button generally implies that the sensor unit is located and applied in an appropriate way such that a reasonable measurement can be taken. A display 12 is provided for displaying e.g. measured values. Since the functions of the sensor unit 1 as proposed in connection with the present embodiment exceed the functions of a conventional sensor unit such as a conventional ear thermometer, a control entity 16 is provided for controlling the functions of the sensor unit 1.

The senior unit 1 can include an identification module 17 the output of which identification 17 module is the entity of an individual in - most likely - some coded form. In one of its simplest embodiments, the identification module 17 is a keyboard for a user to enter the identity of an individual. However, the identification module 17 might comprise some sophisticated sensor for determining the identity of an individual such as any biometrics recognition sensor. The identification module 17 may preferably be part of the sensor unit 1 however, it can also be embodied as a separate unit and transmit its ID data to the sensor unit 1 via a wired or wireless interface.

It is essential for this embodiment not only to measure and display a value representing a physical property of an individual but also to incorporate stored data assigned to this particular individual with the aim to either show such data on the display next to the value measured for better assessing the value measured, e.g. when the data represent values measured on this individual in the past, or to integrate such data into a computation based on the value measured and the data stored - e.g. when the data stored represent a correction value specific to this individual and this correction value is needed to correct the value measured into a corrected value to be displayed to e.g. the medical staff as a user of the sensor unit. Such corrected value or the measured value together with values of the past can form the information displayed on the display.

Consequently, the data stored and assigned to an individual have to be retrieved from the database, in a best mode by querying the database for the identity of the individual. Thus, any identity determined by the identification module 17 is transmitted via respective wireless communication modules 15 and 22 to the database unit 2. Upon receiving such identity data, a database 21 in the database unit 2 can be searched for data assigned to the individual identified by the transmitted identifier. Preferably, a control entity 24 is responsible for extracting the identifier from the message received at the wireless communication module 22, for conducting the query, and for initiating a submission of the data retrieved via the wireless communication module 22 to the sensor unit 1.

The database unit 2 comprises further a wired communications module 23 for communicating e.g. via the Internet or an Intranet. The database unit 2 can be embodied as a server, e.g. for serving this and/or other applications with individual patient data. Such server can be responsible for serving an entire hospital with data, a medical department or a private medical practice. The wireless communication module 22 not necessarily forms part of the database unit 2 itself and can also be embodied elsewhere. However, a wireless path is preferred in the communication between the sensor unit 1 and the database unit 2 as it is one of the major benefits of the embodiment and the present invention as to allow use of a general mobile sensor unit 1 while not abstain from specific data of an individual which individual is subject to the measuremen with the sensor unit 1.

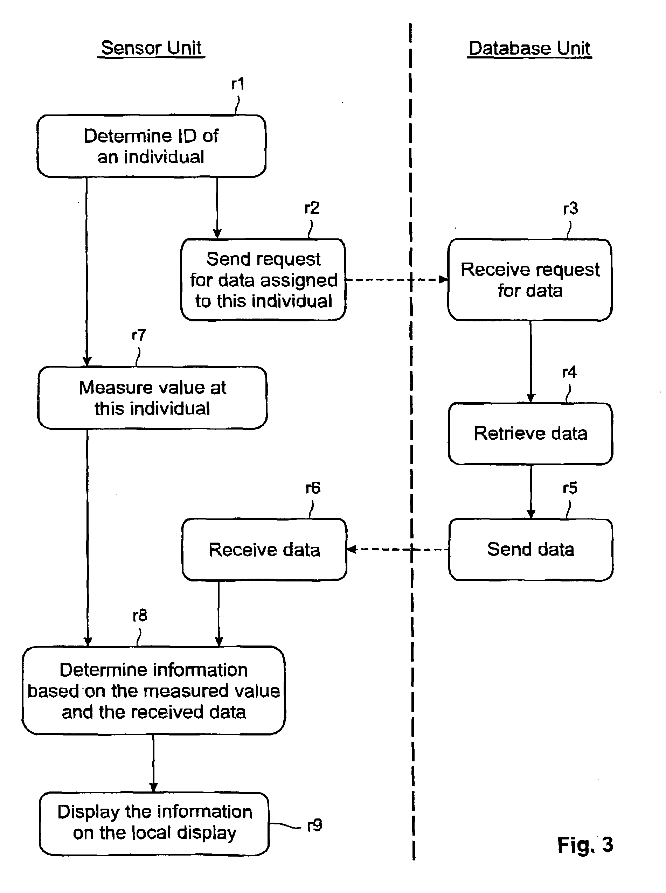

FIG. 3 shows a flow chart of actions assigned to the sensor unit 1 of FIG. 1 on the left hand side, and of actions assigned to the database unit 2 of FIG. 1 on the right hand side. In step r1, in the sensor unit, the identity ID of an individual is determined, e.g. by a user of the sensor unit entering the name of the individual. The identity ID of the individual can be stored at least temporarily. The determination of the identity ID can also be trigger for sending a request for data assigned to this individual to the database unit in step r2. While some of the sensor units can still be used in the conventional way to measure a physical parameter of an individual and displaying the measured value, the determination of the identity ID of an individual can be interpreted as a strong hint that the user wants to include data of this individual which data are stored remote. Thus, once the identity ID of an individual is determined at the sensor unit, this represents a trigger for querying the remote database unit for further data. Once the database unit has received the request - which request of course includes the ID - in step r3, the database is queried for data assigned to the identity ID and thus assigned to the individual represented by the identity ID, to be executed in step r4. As soon as the data are retrieved the data are sent to the sensor unit according to step r5 where they are received in step r6. This data can be transmitted in form of records in a summarized format back to the sensor unit not only in response to a request from that sensor unit but also spontaneously from the back-end to the sensor unit in case the system is designed accordingly.

In the meantime, a physical parameter of the individual person is measured and a corresponding value is received from the sensor, see step r7. Such measurement can be triggered once identity ID of the individual is determined at the sensor unit in step r1 provided the sensor unit is prepared for taking a measurement, such as is arranged appropriately related to the individual. However, it is preferred to have a measurement initiated by the user, e.g. by pushing a start button 14 as depicted in FIG. 1. Consequently, there is no internal time correlation between the point in time the measured value is available and the point in time the data are received from the database unit. Hence, it is preferred to have provided a storage in the sensor unit for storing both of the value measured and the data received. Once both the value measured and the data received are available in the sensor unit, the information to be displayed is determined according to step r8. This can include some computation on the data received and/or on the value measured. Once the information is determined in step r8, the information is displayed on the display of the sensor unit. However, if the information to be displayed consists of the value measured and the data received - this can be the case if the data represent measured values of the past, e.g. the last seven days - neither the data nor the value have to be necessarily stored but can be displayed as soon as available in the sensor unit.

There are many alternative steps included by the scope of the methods according to the invention. In another preferred embodiment, the process is started by first activating the start button of the sensor unit and starting the measurement. Either upon receiving the value measured or upon activating the start button the user can be prompted - e.g. via the display to enter the identity of the individual. In case the identity determination does not need any user interaction but is performed automatically, any such determination process can be triggered the same way.

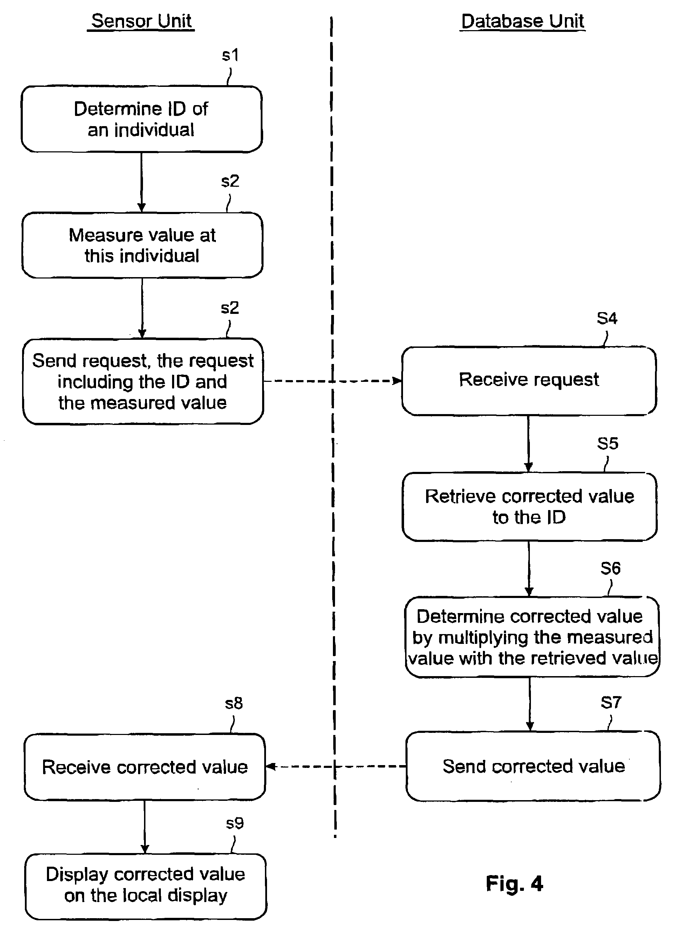

In another flow chart according to FIG. 4, the method described with regard to FIG. 3 is modified by having the identity of the individual in question determined in step s1, and having afterwards measured the value on this individual in step s3. Only if both of the steps s1 and s2 are performed and both the identity of the individual and the value measured are available in the sensor unit, the database unit is approached in step s3. Of course, step s1 and step s2 can be interchanged with respect to their order, and again, the measurement can be triggered by pushing a start button at the sensor unit, the determination of the identity can also be triggered by the user in some appropriate way.

In this particular embodiment, the information to be finally displayed on the display of the sensor unit is determined in the database unit. As in preferred embodiments, such determination comprises rather computational steps based on the measured value and the retrieved data as input than a bare setting of the measured value and the data for display purposes, the processing capabilities of the sensor unit might be undersize. However, the database unit might provide sufficient processing capabilities such that the determination of the information is processed in the database unit. Hence, after having received the request which request then implies the request to determine information based on the value measured and the data to be retrieved from the database unit itself, the value transmitted is preferably stored at least temporanly in the database unit and the data assigned to the individual identified by the transmitted identifier are retrieved from the database in step s5. In this example, the data are represented by a correction value which correction value was stored or computed in the past and represents a value for amending the measured value for this individual. The correction value is thus specific to the individual characterized by the identifyer. In step s6, a corrected value - which simultaneously represents the information to be displayed - is computed by e.g. multiplying the measured value with the correction value. The corrected value is then sent back to the sensor unit, step s7, received there, step s8 and displayed in step s9.

Turning now to FIG. 2, a system is disclosed which comprises an additional component called a mobile hub 3, while the components sensor unit 1 and database unit 2 remain parts of the system. While the elements 11 to 17 of the sensor unit 1 are identical to those depicted and described in connection with the sensor unit 2 in FIG. 1, and the elements 21 to 24 of the database unit 2 are identical to those depicted and described in connection with the database unit 2 in FIG. 1, the wireless connection modules 15 and 22 of sensor unit 1 and database unit 2 are not linked directly, but are linked to counterpart wireless communications modules 34 and 35 of the mobile hub, thereby forming wireless interfaces WI which again preferably represent Bluetooth or WLAN interfaces. In another embodiment, the wireless interface between the mobile hub 3 and the database unit 2 is the interface of a cellular network serving e.g. the GSM standard.

The mobile hub 3 serves as mobile communication platform and can serve many different applications in addition to the one proposed in this embodiment. The mobile hub can be embodied as cell phone or handheld, thereby typically comprising a control entity 31, a display 32, a battery 33, and a keyboard 36. The mobile hub 3 can further connect other peripheral devices to the mobile hub 3 which in turn has connectivity with the cellular network as described above in one example. Through this connection, all these peripheral devices and the sensor unit described in more detail can send and receive data from an Internet server, which Internet server represents e.g. the database unit.

White the physical parameter of the individual is still measured in the sensor unit 1, and the data assigned to the individual are still stored in the database unit 2, the usage of the mobile hub 3 increases flexibility. If the mobile hub 3 comprises a display as the one showed in FIG. 2, this display can make the information based on the value measured and the data stored available. Then, the display 12 of the sensor unit 1 is not even needed. If such display 12 shall still be operated, such display can e.g. show the value measured by the sensor 11.

In case the information to be displayed includes prior processing based on the value measured and the data stored, such processing can now be performed basically at three different locations: the sensor unit 1, the mobile hub 3, and the database 2. The result which represents the information to be displayed is then transmitted to the display of choice. Provided the sensor unit 1 primarily is focussed on measuring a physical parameter, its control entity 16 might not provide sufficient processing capacity for substantial computations. And provided the database unit 2 is also reduced to its basic functions - providing data stored - its control entity 24 might also not be adjusted to process the information needed in an efficient way. Thus, in a very preferred embodiment, the control entity 31 of the mobile hub performs the processing as needed after having received the measured value from the sensor unit and the data stored from the database unit 2.

Again, the mobile hub 3 can also be the preferred component to provide means for determining the identity of the individual. Such identification module 37 can be the module's keyboard 36 in case the identity of an individual can only be entered to the system by typing. However, there can be provided a more sophisticated identification module 37 in addition 10 the keyboard for automatically determining the identity of an individual. In both cases, the provision of any keyboard or other identification module, e.g. identification 17, at the sensor unit 1 becomes redundant.

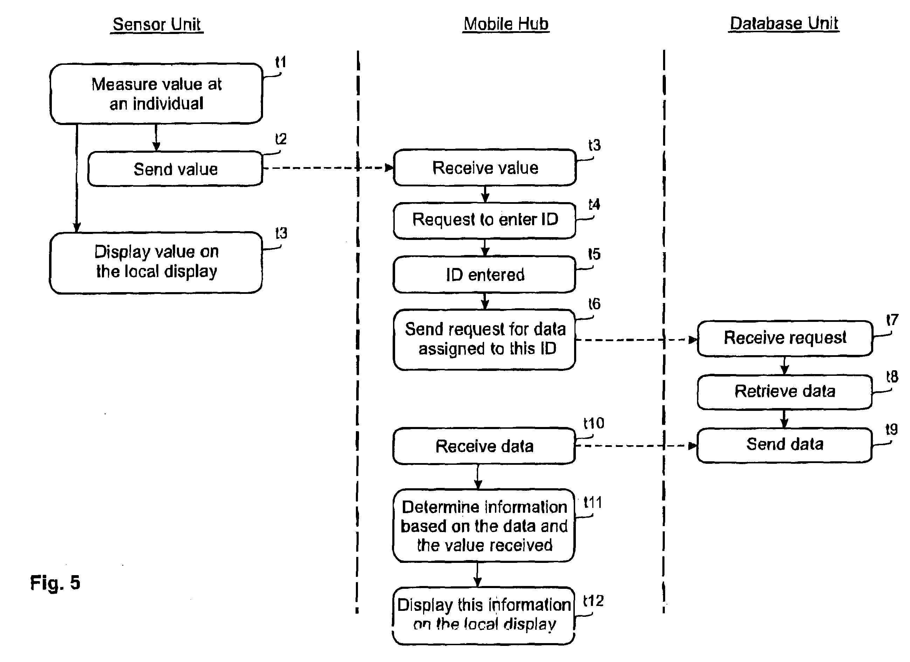

In more detail, a flow chart for displaying information in a system comprising a mobile hub is depicted in FIG. 5.

According to this embodiment, the process is triggered at the sensor unit by measuring a value of a physical parameter of an individual in step t1. Again, such measurement itself is preferably triggered by a user pressing the start button 14 of sensor unit 1. Once the value is available the value is sent via the wireless interface to the mobile hub according to step t3. Provided the sensor unit is equipped with a display, the value can also be shown on this display, see step t13.

Once the value is received at the mobile hub in step t3, this event is taken as a trigger for prompting the user to enter the identity of the individual who was subject to the measurement, step s4, which identity is entered in step t5. Alternatively, an autonomic identity determination can be started in case the mobile hub 3 is provided with such identification module.

Subsequently, a request for providing data stored in the database is submitted to the database unit in step t6, together with the identified identity. As also provided in FIG. 3, the database unit receives the request in step t7, retrieves the data in step t8, e.g. by querying for a patient with the identifier provided, and submits the data retrieved over the wireless connection to the mobile hub in step t9.

There, the data are received in step t10 and the information to be displayed is determined by making use of the value measured and the data received from the database, step t11. Finally, such information is displayed on the hub's display in step t12. Again, the mobile hub can comprise any storage as necessary or needed for storing / caching one or more of the value measured, the data received, and the information to be displayed.

According to this embodiment, the identity determination, the processing of the information to be displayed, and the display of such information is performed in the mobile hub 3. In such configuration, the other units namely the sensor unit 1 and the database unit 3 can concentrate on their core com jetencies which is measuring and data storing respectively. However, as already indicated relation to the sensor unit / database unit only system, any computation can also be performed in the database unit 2 which requires the mobile hub to send also the value measured to the database unit. Then, the information to be displayed on the mobile hub is delivered by the database unit. Both embodiments allow the sensor unit to be embodied as a low cost sensor unit not requiring processing power or display technology. Even the wireles: communications module 15 might be provided only for unidirectional communication towards the mobile hub.

According to another embodiment, it is the display of the sensor unit 1 to be determined to show the information. Here, once the information is determined at the mobile hub, such information is transmitted to the sensor unit to be displayed. Starting from this configuration of the system, and additionally having the information processed at the database unit, the mobile hub more or less forms a platform for connect through any transmittal of information between the sensor unit and the database unit, and vice versa. This configuration can be beneficial whenever the mobile hub is working to full capacity under other applications.

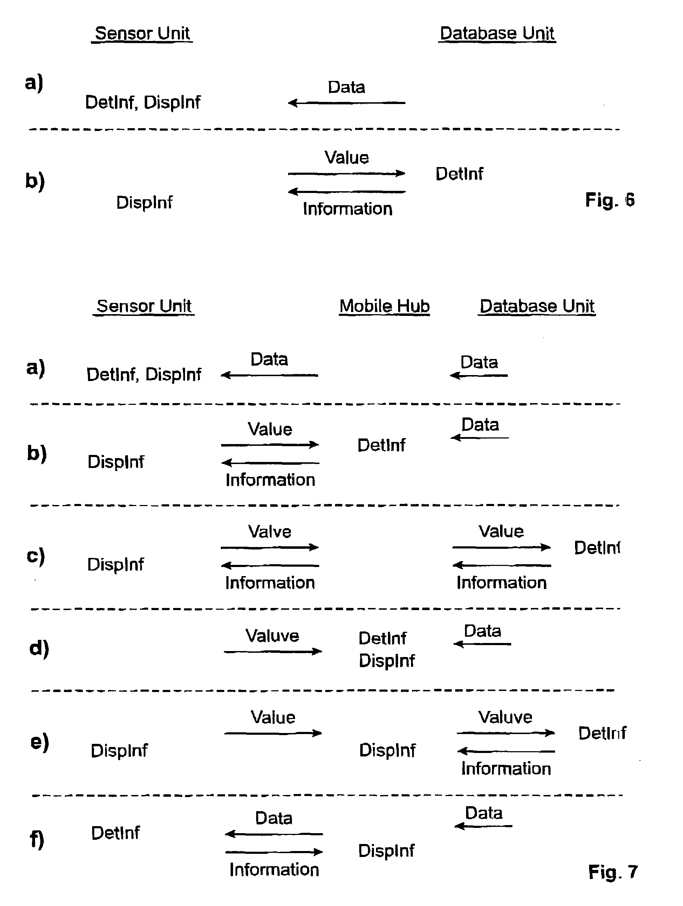

FIG. 6 shows another symbolic diagram of a system comprising a sensor unit and a database unit. While any arrow between the sensor unit and the database unit indicates the flow of "data", "value", and "information" between the units, abbreviations "DetInf" and "DispInf" represent the functions "Determine Information" and "Display Information" respectively. The assignment of these functions indicates the location of executing the function - i.e. the sensor unit or the database unit. The term "value" represents the value measured by the sensor, the term "data" represents the data stored in the database, and the term "information'' represents the information to be determined based on the value and the data and which information is to be displayed. "Data" always has its origin in the database, while "value" always has its origin in the sensor unit.

Thus, in FIG. 6a) a system is shown in which the information is determined and displayed in the sensor unit. In the system according to FIG. 6b), while the information is still shown on the display of the sensor unit, the information is determined in the database unit. Thus, first the value is transmitted from the sensor unit to the database unit, and the information once determined at the database unit is transmitted from the database unit to the sensor unit.

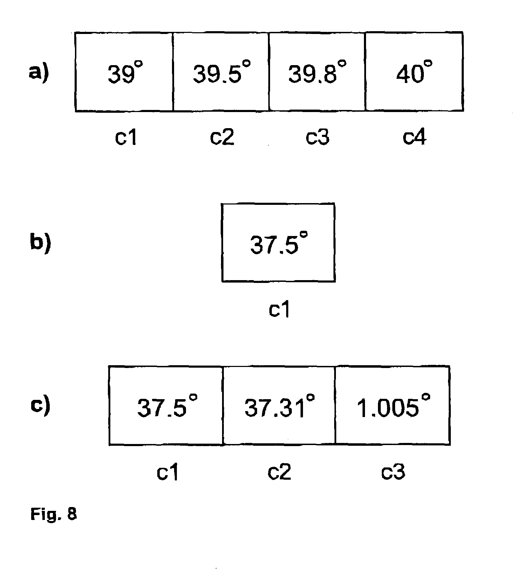

In FIG. 7, the same principles apply as with regard to FIG. 6, however, the system introduced in FIG. 7 additionally comprises a mobile hub and thus represents a system according to FIG. 2.

According to FIG. 7a), the sensor unit gives location for both determining and displaying information, while the mobile hub simply acts as a pass through component for the data provided by the database. In the system according to FIG. 7b), the sensor unit provides the display function, while the mobile hub provides the processing capacity for determining the information to be displayed. Value and data are first submitted to the mobile hub accordingly, while the information once determined is submitted to the sensor unit. In FIG. 7c), the database unit instead provides processing capacity for determining the information. Thus, prior to determining the information the value is transmitted from the sensor unit via the mobile hub to the database unit, while the information determined takes its way back from the database unit to the sensor unit while the sensor unit is still the choice for displaying the information.

In the systems according to FIG. 7c) - 7f), the mobile hub provides the display function. In the system according to FIG. 7d), the value and the data are transmitted to the mobile hub, the information is determined and displayed there. This system is mobile hub centered. The system according to FIG. 7e) differs therefrom in that the determination of information is processed in the database unit. In the system according to FIG. 7f) instead, processing power is provided by the sensor unit.

Finally, FIG. 8 shows different kinds of displays, each having cells c1, c2, .... In FIG. 8a) a display is provided for displaying information which information consists of the value currently measured - displayed in cell c1 - and of data assigned to the same person and stored and provided from the database which data represents the values measured recently on the same person. This data is displayed in display cells c2 - c4. All the values displayed reflect body temperature measured with a thermometer as sensor.

In FIG. 8b) the display displays information in only one cell c1, which information represents a corrected value being computed from a measured value and a correction value. In the display according to FIG. 8c), in cell c1 the corrected value is shown, in cell c2 the measured value is shown, and in cell c3, the correction value is shown which correction value is specific to the person who subject to the measurements. The entirety of values shown build the information displayed.

In this patent application, computer readable program code or program code in the present context mean any expression, in any language, code or notation, of a set of instructions intended to cause a system having an information processing capability to perform a particular function either directly or after either or both of the following a) conversion to another language, code or notation; b) reproduction in a different material form.

While the invention has been described with reference to exemplary embodiments, it will be understood by those skilled in the art that various changes may be made and equivalents may be substituted for elements thereof without departing from the scope of the invention.

## Claims

1. A system for displaying information related to a physical parameter of an individual, comprising
• a sensor (11) for measuring a physical parameter of an individual,
• a database (21) remote from the sensor (11) for storing data assigned to an individual,
• a display (12, 32) remote from the database (21) for displaying the information which information is based on a value measured and provided by the sensor (11) for this individual and based on the data assigned to this individual and provided by the database (21).

2. System according to claim 1,
wherein the data stored in the database (21) and assigned to an individual includes values measured by the sensor (11) in the past.

3. System according to claim 1,
wherein the data stored in the database (21) and assigned to an individual includes an individual correction value for correcting a value measured by the sensor (11).

4. System according to any one of the preceding claims,
wherein the information displayed comprises the value measured and provided by the sensor (11) and the data provided by the database (21).

5. System according to claim 3,
wherein the information displayed includes a corrected value determined by processing the measured value with the correction value.

6. System according to any one of the preceding claims, comprising
a control entity (16, 24, 31) for determining the information.

7. System according to claim 6, comprising
a database unit (2), the database unit (2) comprising the database (21) and the control entity (24).

8. System according to claim 6, comprising
a sensor unit (1), the sensor unit (1) comprising the sensor (11) and the control entity (16).

9. System according to any one of the preceding claims, comprising
a sensor unit (1), the sensor unit (1) comprising the sensor (11) and the display (12).

10. System according to any one of the preceding claims, comprising
a mobile hub (3) comprising a first wireless interface (WI) for communicating to the sensor (11), and a second wireless interface (WI) for communicating to the databas (21).

11. System according to claim 10,
the mobile hub (3) comprising the display (12).

12. System according to claim 10 or claim 11,
the mobile hub (3) comprising the control entity (31).

13. System according to any one of the preceding claims, comprising
means (17, 37) for determining the identity of an individual.

14. Method for displaying information related to a physical parameter of an individual, comprising
• receiving data assigned to the individual from a remote database (21),
• receiving a value measured by a sensor (11) for measuring a physical parameter of the individual,
• displaying the information remote from the database (21), which information is based on the value received from the sensor (11) and on the data received from the database (21).

15. Method for displaying information related to a physical parameter of an individual, comprising
• receiving a value measured by a sensor (11) for measuring a physical parameter of the individual,
• transmitting the value to a remote control entity (24, 31),
• in response to transmitting the value to the control entity (24, 31): receiving information which information is based on the value measured by the sensor (11) and on data assigned to the individual and stored in a remote database (21), and
• displaying the information remote from the database (21).

16. Method for displaying information related to a physical parameter of an individual, comprising
• receiving a value measured by a sensor (11) for measuring a physical parameter of the individual,
• transmitting the value to a remote control entity (24, 31),
• in response to transmitting the value to the control entity (24, 31): receiving information which information is based on the value measured by the sensor (11) and on data assigned to the individual and stored in a remote database (21), and
• transmitting the information to a display (12, 32) remote from the database (21).

17. Method for displaying information related to a physical parameter of an individual, comprising
• receiving a value measured by a sensor (11) for measuring a physical parameter of the individual,
• receiving data assigned to the individual from a remote database (21),
• determining the information which information is based on the value measured by the sensor (11) and on data assigned to the individual and stored in a remote database (21), and
• transmitting the information to a display (12, 32) remote from the database (21).

18. Method according to claim 14, comprising
determining the information prior to displaying it.

19. Method according to any one of the preceding claims 14 to 18, comprising
performing the steps of the method in a sensor unit (1) comprising the sensor (21).

20. Method according to any one of the preceding claims 14 to 18,
performing the steps of the method in a mobile hub (3), the mobile hub (3) being designed for wireless communicating to the sensor (11) and to the database (21).

21. A computer program element comprising program code for performing the steps of the method of any of the claims 14 to 17 when said program code is run on a processing unit.

22. A sensor unit, comprising
a control entity designed for performing steps according to a method as claimed in any one of the claims 14 to 18.

23. A mobile hub, comprising
a control entity designed for performing steps according to a method as claimed in any one of the claims 14 to 18.
